(19) Europäisches Patentamt
European Patent Office
Office européen des brevets

(11) EP 1 132 382 A1

(12) # EUROPEAN PATENT APPLICATION

(43) Date of publication:
**12.09.2001 Bulletin 2001/37**

(51) Int Cl.[7]: **C07D 285/32**, A61K 31/549, A61P 9/12

(21) Application number: **00200825.8**

(22) Date of filing: **08.03.2000**

(84) Designated Contracting States:
**AT BE CH CY DE DK ES FI FR GB GR IE IT LI LU MC NL PT SE**
Designated Extension States:
**AL LT LV MK RO SI**

(71) Applicant: **Farma Invent B.V.**
**6533 NV Nijmegen (NL)**

(72) Inventor: **Guelen, Petrus Johannes Maria**
**5371 KE Ravenstein (NL)**

(74) Representative: **Ottevangers, Sietse Ulbe et al**
**Vereenigde,**
**Postbus 87930**
**2508 DH Den Haag (NL)**

(54) **3-Oxy-7-sulfamyl-3,4-dihydro-1,2,4-benzothiadazine-1,1-dioxide derivatives having diuretic and/or natriuretic activity**

(57) Compound of the formula

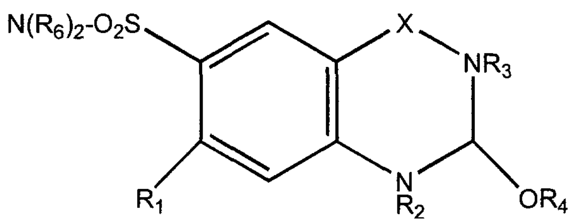

wherein

$R_1$ is H, Cl, Br, F, trifluoromethyl, trichloromethyl, $C_1$-$C_5$-alkyl or $C_3$-$C_7$-cycloalkyl, optionally substituted with one or more hydroxy groups, halogen atoms, trifluoromethylgroups, amino or substituted aminogroups, nitrogroups or alkoxy or aryloxygroups, $C_1$-$C_5$-alkoxy, optionally substituted with one or more hydroxy groups, halogen atoms, trifluoromethylgroups, amino or substituted aminogroups, nitrogroups or alkoxy or aryloxygroups, $NO_2$, $NH_2$;
X is $SO_2$, C=O, C=S;
$R_2$ and $R_3$ are independently H, $C_1$-$C_5$-alkyl or a $C_3$-$C_7$-cycloalkyl;
$R_4$ is H; $C_1$-$C_{10}$-alkyl (examples of which are methyl, ethyl, propyl, butyl, pentyl, hexyl, heptyl, octyl, nonyl, decyl and isomers thereof); $C_1$-$C_{10}$-cycloalkyl; allyl; $C_1$-$C_{10}$-aryl (examples of which are optionally substituted phenyl, tolyl, xylyl) or heteroaryl; benzyl; $C_1$-$C_{10}$-alkenyl; $C_1$-$C_{10}$-(cyclo)alkenyl; $C_1$-$C_{10}$-ester; $C_1$-$C_{10}$-amide; polyethylene glycol ethers with 1 to 10 glycolunits; $-C(O)N(R_5)_2$;$R_5$ is $C_1$-$C_5$-alkyl or $C_3$-$C_7$-cycloalkyl, optionally substituted with one or more hydroxy groups, halogen atoms, trifluoromethylgroups, amino or substituted aminogroups, nitrogroups or alkoxy or aryloxygroups, $C_1$-$C_5$-alkoxy optionally substituted with one or more hydroxy groups, halogen atoms, trifluoromethylgroups, amino or substituted aminogroups, nitrogroups or alkoxy or aryloxygroups, $NO_2$, $NH_2$;
$R_6$ is independently H, trifluoromethyl, trichloromethyl $C_1$-$C_5$-alkyl or $C_3$-$C_7$-cycloalkyl, optionally substituted with one or more hydroxy groups, halogen atoms, trifluoromethylgroups, amino or substituted aminogroups, nitrogroups or alkoxy or aryloxygroups, $C_1$-$C_5$-alkoxy optionally substituted with one or more hydroxy groups, halogen atoms, trifluoromethylgroups, amino or substituted aminogroups, nitrogroups or alkoxy or aryloxygroups; and

salts, hydrolysable esters and/or solvates thereof.



Printed by Jouve, 75001 PARIS (FR)

## Description

The present invention is directed to certain hydroxyhydrothiazides and related compounds, to a process for the preparation of these hydroxyhydrothiazides and related compounds, to a pharmaceutical composition comprising hydroxyhydrothiazides and related compounds, to the use of hydroxyhydrothiazides and related compounds for the preparation of a medicament having diuretic and/or natriuretic activity and to the use of hydroxyhydrothiazides and related compounds for the preparation of a medicament for the treatment of decompensatio cordis, oedema and/or hypertension or related disorders, or disorders associated therewith.

Thiazides are a class of chemical compounds widely recognised for their diuretic activity. The diuretic activity of thiazides have resulted in their application in pharmaceutical compositions having diuretic activity. Pharmaceutical compositions comprising these active thiazides are used, among others, in the treatment of decompensatio cordis, oedema and hypertension.

Thiazide-based diuretics and anti-hypertensives are widely known, for instance in the form of compositions comprising benzthiazide(6-chloro-3-(((phenylmethyl)thio) methyl)-2H-1,2,4-benzothiadazine-7-sulfonamide-1,1-dioxide) (see e.g. Merck index $12^{th}$ Ed. For a large number of thiazides). Thiazide-based compounds and the application of these compounds in pharmaceutical compositions are also disclosed in US-A 2,886,566. This patent describes 3-oxo-7-sulfamyl-3,4-dihydro-1,2,4-benzothiadazine-1,1-dioxides. The compounds of this US patent are considered to be pharmaceutical agents having diuretic properties which make them useful for in the treatment of hypertension or congestive heart failure and other disorders that produce oedematous conditions in the body.

Other thiazide based compounds are described in US-A-3,025,292 (hydrochlorothiazide) and US-A-2,809,194 (chlorothiazide).

A group of pharmaceutically active compounds that are pharmaceutically and chemically related to thiazides are known and depicted in the art as thiazide-like drugs or diuretics. See in this respect Goodman & Gilman's " The pharmacological basis of therapeutics" $9^{th}$ Edition, McGraw Hill, 1995, pages 701-703.

In these compounds the $SO_2$-group in the benzothiadazine-1,1-dioxide is replaced by a C=O (carbonyl)-functionality. These compounds exhibit thiazide-like diuretic behaviour. Examples of these thiazide-like drugs are metolazone (7-chloro-1,2,3,4-tetrahydro-2-methyl-4-oxo-3-*o*-tolyl-6-quinazoline sulfonamide) and quinethazone (7-chloro-2-ethyl-1,2,3,4-tetrahydro-4-oxo-6-quinazoline sulfonamide).

The thiazide as well as thiazide-like diuretics act by inhibiting sodium and chloride co-transport across the luminal membrane of the early segment of the distal tubular cells in the kidney. Most side effects associated with thiazide diuretics are dose related and can be minimised with low dose therapy.

The literature in general is silent with respect to the metabolism of the thiazide diuretics. No metabolites have been described in the literature and it is generally assumed that the thiazides are excreted in unchanged (parent) form. There is no indication in the prior art that not the thiazide-based compounds per se but metabolites thereof would be responsible for the diuretic activity. Rather the contrary is found: the vested literature such as "Drug level monitoring", John Wiley and Sons, Volume II Edited by E.T. Lin and W. Sadée in Chapter 14 by V.M. Knepp on page 286-291; Goodman & Gilman's "The pharmacological basis of therapeutics" $9^{th}$ Edition, McGraw Hill, 1995 pages 701-703; "Disposition of Toxic drugs and chemicals in man", $2^{nd}$ Ed. R.C. Baselt Ph.D., 1982, Biomedical publications, Davis, Ca. USA pages 380-381; Handbook of experimental pharmacology, Springer Verlag, 1969, Band XXIV, pages 266-27 stipulate that the thiazide parent compound is mainly eliminated unchanged through renal excretion. No further data are known at present on the metabolism of the thiazide diuretics.

There remains a continuous need for compounds with improved diuretic and anti-hypertensive activities for the application in pharmaceuticals to come to drugs that have an improved performance. The improved performance of a pharmaceutical composition in general relates to the improvement of properties such as activity and selectivity. Generally these properties can be translated in terms of effectiveness such as the amount that has to be taken by a patient and the effective duration of a single dose.

When studying the bioavailability of epithiazide in healthy volunteers, the present inventor has surprisingly found that, after administration of a suitable dose of epithiazide, the compound was detected in the urine, but that in the plasma no epithiazide could be detected. A compound with a longer biological half-life was found. This compound has been synthesised in the laboratory and was identified as hydroxyhydrothiazide. Further studies revealed that hydroxyhydrothiazides were also formed upon hydrolysis of other thiazide derivatives such as hydrochlorothiazide, epithiazide and others. It was also found that the thiazide-like drugs expressed similar hydrolysis behaviour as thiazides in this respect.

The invention relates to the novel class of thiazide compounds that have an enhanced diuretic and/or natriuretic activity.

The invention accordingly relates to compounds of the formula:

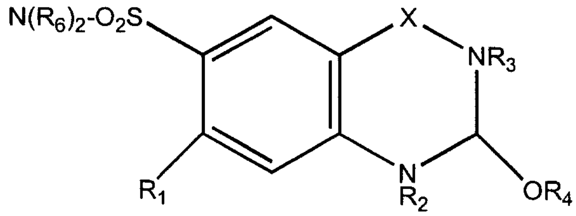

wherein

$R_1$ is H; Cl; Br; F; trifluoromethyl; trichloromethyl; $C_1$-$C_5$-alkyl or $C_3$-$C_7$-cycloalkyl, which alkyl or cycloalkyl are optionally substituted with one or more hydroxy groups, halogen atoms, trifluoromethylgroups, amino or substituted aminogroups, nitrogroups or alkoxy or aryloxygroups; $C_1$-$C_5$-alkoxy, optionally substituted with one or more hydroxy groups, halogen atoms, trifluoromethylgroups, amino or substituted aminogroups, nitrogroups or alkoxy or aryloxygroups; $NO_2$; $NH_2$;
X is $SO_2$, C=O or C=S;
$R_2$ and $R_3$ are independently H, $C_1$-$C_5$-alkyl or a $C_3$-$C_7$-cycloalkyl;
$R_4$ is H; $C_1$-$C_{10}$-alkyl (examples of which are methyl, ethyl, propyl, butyl, pentyl, hexyl, heptyl, octyl, nonyl, decyl and isomers thereof); $C_1$-$C_{10}$-cycloalkyl; allyl; $C_1$-$C_{10}$-aryl (examples of which are optionally substituted phenyl, tolyl, xylyl) or heteroaryl; benzyl; $C_1$-$C_{10}$-alkenyl; $C_1$-$C_{10}$-(cyclo)alkenyl; $C_1$-$C_{10}$-ester; $C_1$-$C_{10}$-amide; polyethylene glycol ethers with 1 to 10 glycolunits; $-C(O)N(R_5)_2$;
$R_5$ is $C_1$-$C_5$-alkyl or $C_3$-$C_7$-cycloalkyl, optionally substituted with one or more hydroxy groups, halogen atoms, trifluoromethylgroups, amino or substituted aminogroups, nitrogroups or alkoxy or aryloxygroups, $C_1$-$C_5$-alkoxy optionally substituted with one or more hydroxy groups, halogen atoms, trifluoromethylgroups, amino or substituted aminogroups, nitrogroups or alkoxy or aryloxygroups, $NO_2$, $NH_2$;
$R_6$ is independently H, trifluoromethyl, trichloromethyl $C_1$-$C_5$-alkyl or $C_3$-$C_7$-cycloalkyl, optionally substituted with one or more hydroxy groups, halogen atoms, trifluoromethylgroups, amino or substituted aminogroups, nitrogroups or alkoxy or aryloxygroups, $C_1$-$C_5$-alkoxy optionally substituted with one or more hydroxy groups, halogen atoms, trifluoromethylgroups, amino or substituted aminogroups, nitrogroups or alkoxy or aryloxygroups; and
salts, hydrolysable esters and/or solvates thereof. Preferably $R_1$ is $CH_3$ , $CF_3$ or Cl; both $R_2$ and $R_3$ are independently H, $CH_3$ or (cyclo) alkyl; X is $SO_2$, C=O, more preferably $SO_2$; $R_4$ is H and $R_6$ is H, trifluoromethyl, trichloromethyl, $C_1$-$C_5$-(cyclo)alkyl. More preferably $R_1$ is Cl; X is $SO_2$; $R_2$, $R_3$, $R_4$ and $R_6$ are H.

In a second aspect of the invention provides for pharmaceutical compositions comprising at least one compound according to the invention and pharmaceutically acceptable salts, hydrolysable esters and/or solvates thereof.

In another aspect of invention, the hydroxy functionality at the C-3 position is further derivatised, preferably with a group that is easily cleaved, preferably in situ. Suitable groups with which the hydroxy functionality can be derivatised are alkyl-, allyl-, aryl-, alkenyl-ether or -ester with 1 to 10 carbon atoms, amide, $-C(O)N(R_5)_2$, wherein $R_5$ is $C_1$-$C_5$-alkyl or $C_3$-$C_7$-cycloalkyl, optionally substituted with one or more hydroxy groups, halogen atoms, trifluoromethylgroups, amino or substituted aminogroups, nitrogroups or alkoxy or aryloxygroups, $C_1$-$C_5$-alkoxy optionally substituted with one or more hydroxy groups, halogen atoms, trifluoromethylgroups, amino or substituted aminogroups, nitrogroups or alkoxy or aryloxygroups, $NO_2$, $NH_2$, preferably with ester, amide or $-C(O)N(R_5)_2$ groups. These derivatives of the compounds according to the invention may provide for additionally improved properties of the resulting pharmaceutical compositions. Such improved properties can be of a pharmacokinetic nature but also properties as stability and consequently storability can be improved. The derivatised hydroxycompounds according to the invention can be used as precursors. The derivatised compounds may have a lower renal excretion. When combined with low rate of hydrolysis to the active hydroxyderivative, these compounds may provide for pharmaceutical compositions that have a prolonged activity. The compounds according to the invention can be used in a derivatised form (pro-drug). Such pro-drugs are subsequently transformed in the body in the pharmaceutically desired form without adverse effects.

To this end preferred derivatised compounds according to the invention are those that are derivatised in such manner that the rate of excretion of the derivatised compound is lower than the excretion rate of the corresponding hydroxy derivative. Also the metabolising rate of the derivatised thiazide towards the corresponding hydroxy derivative is lower. This enables the formulation of preferred pharmaceutical compositions that provide the body with a more constant dose over a prolonged period of time. This enables the formulation of compositions that either need to be taken less frequently or that contain less of the pharmaceutically active compound.

The compositions according to the invention can be in the form of pharmaceutically acceptable salts, esters

and/or solvate of the compounds according to the invention, preferably in the form of a sodium or a potassium salt of the compounds according to the invention. Preferably, a composition according to the invention can further be formulated with binders, diluents, desintegrants, gliders, humectants, surfactants, colouring and flavouring agents and other additives known in the art and used as such.

Suitable desintegrants may be selected from alginates, carboxymethylcelluloses, siliciumdioxide, croscarmellose, crospovidone, guar gum, magnesium aluminium silicate, cellulose and derivatives thereof, starch and modified starch, cationic resins glycollates, glycinates and combinations, salts and mixtures thereof.

Suitable diluents are for instance carbohydrate diluents such as sugars such as compressible sugar confectioners sugar, dextrates, dextrose, fructose, cellulose, gelatinised starch, lactose, maltodextrine, mannitol, sorbitol, sucrose, lactitol, maltitol or xylitol or mixtures thereof.

The compositions according to the invention provide for pharmacotherapeutic agents that have an improved diuretic capacity and a longer half-life. The half life of the novel compounds has been found to be considerably longer, e.g. twice as long when compared with conventionally known thiazide and thiazide-like diuretics such as epithiazide and hydrochlorothiazide.

Such an increase in half life of a pharmaceutically active compound has a number of profound therapeutical and economical consequences which make the compounds according to the invention attractive alternatives for the known diuretics. One of the advantages is that the prolonged half-life allows for administration to a patient only once a day instead of , e.g., twice. Another advantage of the increased capacity is that lower doses are needed for achieving the same diuretic capacity.

The pharmaceutical compositions according to the invention result in an increase of the excretion of water and of salt urinary excretion. These compositions may suitably be used in the treatment of oedema, hypertension or decompensatio cordis and related disorders or disorders associated herewith.

The pharmaceutical compositions according to the invention may further comprise other compounds that result in the desired diuretic or natriuretic characteristics. Such combinations can be made with betablockers, compounds or compositions that prevent the depletion of potassium such as potassium retainers, or vascular active compounds to further enhance the effectiveness of the compositions according to the invention. Examples of compositions that can suitably be combined with the compositions according to the invention are amiloride, benazepril, bisoprolol, captopril, enalapril, guanethidine, hydralazine, lisinopril, losartan, methyldopa, metoprolol moexipril, propranolol, reserpine, spironolactone, timolol, triamterene, valsartan.

The compounds according to the invention and preferable the hydroxyhydrothiazides according to the invention have been found effective over a wide dosage range. Suitable dosages are in the range of 0.001 g/kg up to 15 mg/kg body weight, preferably from 0.05 to 5 mg/kg, more preferably from 0.01 to 1 mg/kg. The most preferred range is 0.01 mg/kg to 0.1 mg/kg, with 1-2 mg as the preferred effective dose.

The dosages in which the compounds according to the invention can be suitably administered are very low when compared with the average $LD_{50}$ value for comparable compounds, which was found to be up to 600 mg/kg in mice (orally).

The compositions according to the invention can be administered in therapeutic dosages in conventional vehicles. They can be administered in the form of, e.g. tablets, capsules for oral administration. Also the parental, intramuscular, rectal, nasal or transdermal administration of the compounds leads to advantageous effects of the new compounds. The previously mentioned administration forms include long acting forms such as slow- or controlled release formulations.

The compositions according to the invention can be dissolved, dispersed or emulsified in suitable solvents, dispersants or carrier materials to further aid in the provision of administrable pharmaceutical compositions.

In a preferred embodiment the pharmaceutical compositions according to the invention are in a form suitable for oral, rectal, parenteral, nasal and transdermal application.

As mentioned here-in-above, the pharmaceutical compositions according to the invention generally have a half-life that is considerably longer than that of conventionally known thiazides. A preferred form of administration is therefor a direct release formulation. However, the pharmaceutical compositions according to the invention may also suitably be provided in the form of controlled release formulations.

A further aspect of the invention pertains to a process for the preparation of the compounds according to the invention. The compounds according to the invention and preferably the hydroxythiazides according to the invention are prepared by acidic, basic or enzymatic hydrolysis of suitable thiazide precursors which are described in the literature and often are commercially available. In general this means that a suitable precursor is any thiazide or thiazide-like compound that allows for the hydrolysis at the C-3 position. It has been found that when these compounds are subjected to aqueous reactions conditions, preferably in the presence of a buffer, this hydrolysis readily takes place.

The compounds according to the invention and preferably the hydroxyhydrothiazides according to the invention are obtained through hydrolysis at the C-3 position of known thiazides under acidic, alkaline or enzymatic conditions. The hydrolysis is successfully carried out over a wide temperature and pH range.

The compositions according to the present invention have a stronger potassium retaining activity than conventionally known thiazide or thiazide-like compounds containing compositions. The stronger potassium retaining activity reduces the risk of hyperkalaemia. This is a considerable advantage over the known thiazide-based or -like diuretics and hypertensives as the reduced risk of hyperkalaemia provides for an enhanced safety during long term treatment with pharmaceutical compositions comprising compounds according to the present invention.

In a preferred embodiment the compounds according to the invention have an increased natriuretic activity.

An aspect of the invention is therefore the use of at least one compound according to the invention and pharmaceutically acceptable salts, hydrolysable esters and/or solvates thereof for the preparation of a medicament having a diuretic and/or natriuretic activity and/or for the treatment of hypertension and/or decompensatio cordis and related disorders, or disorders associated herewith.

Description of the figure:

Figure 1: Hydrolysis of epithiazide to hydroxyhydrothiazide under various temperatures and pH in a 0.067 M phosphate buffer.

Further the invention will be described in more detail while referring to the following non-limiting examples.

Example 1.

Epithiazide was contacted with an aqueous phosphate buffer of 0.067 M at two different temperatures (RT and 37°C), at different pHs, ranging from 5,2 to 13. At pH=7 and 37°C, the epithiazide was cleaved for more than 80 % in 10 hours into the desired hydroxyhydrothiazide. The results are given in Fig 1.

The hydroxyhydrothiazide was synthesised by the hydrolysis of epithiazide. Conversion to hydroxyhydrothiazide was completed in 72 hours at 37 °C in a 0.067 M phosphate buffer. After isolation and purification, the product hydroxyhydrothiazide was a light brown to white powder with a purity of > 99.5 % (HPLC, UV detection at 272 nm), pKa =9.5, Mp 160-162 °C; UV(1 cm, 260 nm, 0.1 M NaOH) E=502.

Example 2

The pharmacotherapeutic activity of the hydroxyhydrothiazides has been evaluated. The natriuretic an/or diuretic activity has been determined by testing on animals and healthy volunteers.

Example 2A

Intravenous administration of hydroxyhydrothiazide to dogs in a dosage of 0.08-0.4 mg/kg resulted in the rapid distribution of the compound throughout the body. The elimination from the body was monitored for 48 hours. It was found that the half-life was about twice as long when compared with conventional thiazide-based diuretics such as epithiazide and hydrochlorothiazide. The diuretic effect was monitored by measuring the urine production. It was found that an instant diuretic effect was noted upon administration of the compounds according to the invention.

Example 2B

Oral administration to healthy male volunteers in a dose of 2 mg (about 0.08 mg/kg) also resulted in an increase of the urinary flow. The urinary flow was measured over a period of 24 hours prior to the oral administration of the compounds of the present invention up to 72 hours after administration. The average urinary flow was found to be 1 ml/min. Determination of the urinary flow after drug administration resulted, over a period of 24 hours, in an average urinary flow of 2 ml/min, with peak flows of 5 ml/min. The drug was detected in the urine of volunteers until 72 hours after administration. This indicates an increased half-life over the known thiazide diuretics. The natriuretic properties of the compounds according to the invention were determined by measuring the sodium content in the urine post and pre-dose. It was found that the concentration of sodium post dose was markedly increased.

## Claims

**1.** Compound of the formula

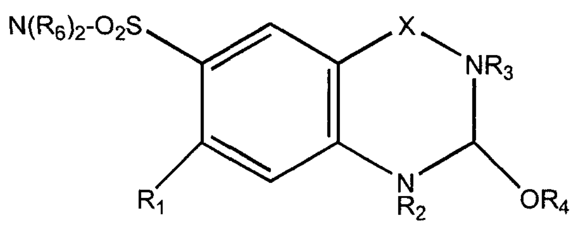

wherein

$R_1$ is H, Cl, Br, F, trifluoromethyl, trichloromethyl, $C_1$-$C_5$-alkyl or $C_3$-$C_7$-cycloalkyl, optionally substituted with one or more hydroxy groups, halogen atoms, trifluoromethylgroups, amino or substituted aminogroups, nitrogroups or alkoxy or aryloxygroups, $C_1$-$C_5$-alkoxy, optionally substituted with one or more hydroxy groups, halogen atoms, trifluoromethylgroups, amino or substituted aminogroups, nitrogroups or alkoxy or aryloxygroups, $NO_2$, $NH_2$;
X is $SO_2$, C=O, C=S;
$R_2$ and $R_3$ are independently H, $C_1$-$C_5$-alkyl or a $C_3$-$C_7$-cycloalkyl;
$R_4$ is H; $C_1$-$C_{10}$-alkyl; $C_1$-$C_{10}$-cycloalkyl; allyl; $C_1$-$C_{10}$-aryl or heteroaryl; benzyl; $C_1$-$C_{10}$-alkenyl; $C_1$-$C_{10}$-(cyclo) alkenyl; $C_1$-$C_{10}$-ester; $C_1$-$C_{10}$-amide; polyethylene glycol ethers with 1 to 10 glycolunits; $-C(O)N(R_5)_2$;
$R_5$ is $C_1$-$C_5$-alkyl or $C_3$-$C_7$-cycloalkyl, optionally substituted with one or more hydroxy groups, halogen atoms, trifluoromethylgroups, amino or substituted aminogroups, nitrogroups or alkoxy or aryloxygroups, $C_1$-$C_5$-alkoxy optionally substituted with one or more hydroxy groups, halogen atoms, trifluoromethylgroups, amino or substituted aminogroups, nitrogroups or alkoxy or aryloxygroups, $NO_2$, $NH_2$;
$R_6$ is independently H, trifluoromethyl, trichloromethyl $C_1$-$C_5$-alkyl or $C_3$-$C_7$-cycloalkyl, optionally substituted with one or more hydroxy groups, halogen atoms, trifluoromethylgroups, amino or substituted aminogroups, nitrogroups or alkoxy or aryloxygroups, $C_1$-$C_5$-alkoxy optionally substituted with one or more hydroxy groups, halogen atoms, trifluoromethylgroups, amino or substituted aminogroups, nitrogroups or alkoxy or aryloxygroups; and
salts, hydrolysable esters and/or solvates thereof.

2. Compound according to claim 1, wherein $R_1$ is $CH_3$, $CF_3$ or Cl; both $R_2$ and $R_3$ are independently H, $CH_3$ or (cyclo) alkyl; $R_4$ is H and $R_6$ is H, trifluoromethyl, trichloromethyl, $C_1$-$C_5$-alkyl or $C_3$-$C_7$-cycloalkyl and X is $SO_2$ or C=O.

3. Compound according to claim 1 or 2, wherein R1 is Cl; $R_2$, $R_3$, $R_4$ and $R_6$ are H and X is $SO_2$.

4. Compound according to claims 1-3, wherein the pharmacologically acceptable salt is a sodium or potassium salt.

5. Pharmaceutical composition comprising at least one compound as defined in claim 1 and pharmaceutically acceptable salts, hydrolysable esters and/or solvates thereof.

6. Pharmaceutical composition according to claim 5 in a form suitable for oral, rectal, parenteral, nasal and transdermal application.

7. Process for the preparation of compounds as defined in claim 1 comprising a step in which a thiazide precursor or a precursor for a thiazide-like drug is subjected to aqueous conditions for a period sufficiently long to effectively hydrolyse the C-3 position.

8. Use of at least one compound as defined in claim 1 and pharmacologically acceptable salts esters and solvates thereof for the preparation of a medicament having diuretic or natriuretic activity.

9. Use of at least one compound as defined in claim 1 and pharmacologically acceptable salts esters and solvates thereof for the preparation of a medicament having a diuretic and/or natriuretic activity and/or for the treatment of hypertension and/or decompensatio cordis and related disorders.

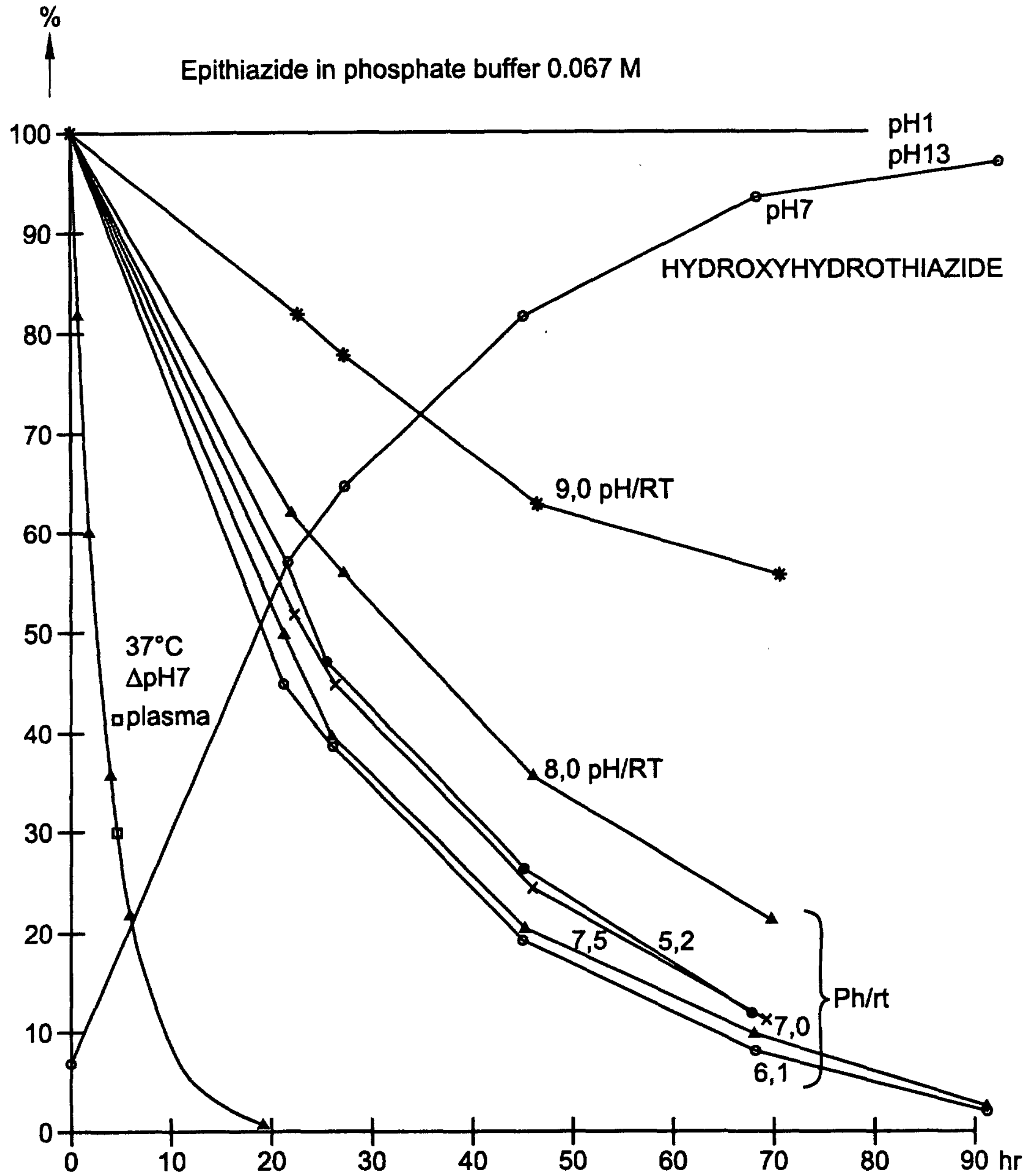
%
Epithiazide in phosphate buffer 0.067 M
100
90
80
70
60
50
40
30
20
10
0
pH1
pH13
pH7
HYDROXYHYDROTHIAZIDE
9,0 pH/RT
37°C
ΔpH7
plasma
8,0 pH/RT
7,5
5,2
Ph/rt
7,0
6,1
0
10
20
30
40
50
60
70
80
90 hr

FIG. 1

European Patent Office

# EUROPEAN SEARCH REPORT

Application Number
EP 00 20 0825

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y,D | US 3 025 292 A (JONES WILLIAM H.) 13 March 1962 (1962-03-13) * column 2, line 27 - line 56 * --- | 1-9 | C07D285/32 A61K31/549 A61P9/12 |
| Y | US 3 458 513 A (SHETTY BOLA VITHAL) 29 July 1969 (1969-07-29) * column 5, line 61 - line 63; claims 1-3 * --- | 1-9 | |
| A,D | US 2 886 566 A (NOVELLO C. FREDERICK) 12 May 1959 (1959-05-12) * column 1, line 70 - line 73; claim 1 * ----- | 1-9 | |

TECHNICAL FIELDS SEARCHED (Int.Cl.7)
C07D

The present search report has been drawn up for all claims

| Place of search | Date of completion of the search | Examiner |
|---|---|---|
| MUNICH | 26 July 2000 | Seymour, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 20 0825

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-2000

| Patent document cited in search report | Publication date | Patent family member(s) | Publication date |
|---|---|---|---|
| US 3025292 A | | NONE | |
| US 3458513 A | 29-07-1969 | NONE | |
| US 2886566 A | | NONE | |

EPO FORM P0459

For more details about this annex : see Official Journal of the European Patent Office, No. 12/82